# FASCICULE DE BREVET EUROPEEN

(11) **EP 1 406 616 B1**
(45) Date de publication et mention de la délivrance du brevet: **12.09.2007**
(21) Numéro de dépôt: 02747627.4
(22) Date de dépôt: 05.07.2002
(51) Int. Cl.: A61K 31/409, A61K 9/08, A61P 35/00

(54) **FORMULATION GALENIQUE INJECTABLE POUR UTILISATION DANS UN DIAGNOSTIC OU UNE THERAPIE PHOTODYNAMIQUE ET SON PROCEDE DE PREPARATION**
INJIZIERBARE GALENISCHE FORMULIERUNG ZUR VERWENDUNG IN EINER PHOTODYNAMISCHEN DIAGNOSE ODER THERAPIE SOWIE IHR HERSTELLUNGSVERFAHREN
INJECTABLE GALENIC FORMULATION FOR USE IN A DIAGNOSIS OR A PHOTODYNAMIC THERAPY AND METHOD FOR PREPARING SAME

(30) Priorité: 17.07.2001 EP 01810708
(43) Date de publication de la demande: 14.04.2004
(73) Titulaire: Steba Biotech N.V., 2585 GB Den Haag (NL)
(72) Inventeur: BRUN, Pierre-Hervé, F-75019 Paris (FR); PRUDHOMME, Alain, F-29940 La Forêt Fouesnant (FR); BARNOUX, Jean-Luc, F-25160 Chaon (FR)
(74) Mandataire: Reuteler, Raymond Werner
(86) Numéro de dépôt international: PCT/IB2002/002661
(87) Numéro de publication internationale: WO 2003/009842

(56) Documents cités:
- WO-A-00/33833
- US-A- 5 462 726

## Description

La présente invention concerne une formulation galénique injectable pour utilisation dans un diagnostic ou une thérapie photodynamique (PDT) contenant un agent photosensibilisant dérivé d'une bactériochlorophylle modifiée et un véhicule en phase aqueuse pharmaceutiquement acceptable.

La présente invention concerne également un procédé de préparation de cette formulation galénique injectable.

Il y a plus d'une cinquantaine d'années, il a pu être démontré que des porphyrines pouvaient s'accumuler dans des tissus tumoraux et de plus qu'elles pouvaient fluorescer dans ces tissus tumoraux irradiés en absorbant la lumière in situ.

En raison de leurs propriétés, ces porphyrines se sont donc révélées constituer un moyen intéressant pour détecter une tumeur par localisation de la fluorescence.

Ces dernières années, l'utilisation d'agents photosensibilisants dans le traitement du cancer a fait l'objet de nombreuses études qui se sont révélées très prometteuses de sorte que l'intérêt de cette technique connue sous le nom de thérapie photodynamique (PDT) ne cesse de croître.

Dans cette technique de thérapie photodynamique (PDT), on administre un agent photosensibilisant qui est capable de se fixer préférentiellement dans les tissus où les cellules prolifèrent et on illumine ces tissus avec une lumière ayant une longueur d'onde qui soit absorbée par le produit photosensibilisant au moyen d'une lumière monochromatique (laser) ou polychromatique (lampe).

Le produit photosensibilisant, qui n'est pas toxique en lui-même, a la propriété, après avoir été excité par l'absorption de photons, de revenir à son état de base en transférant l'énergie vers l'oxygène ambiant sous la forme d'oxygène singulet (photooxydation de type II) et/ou de radicaux libres oxygénés (photooxydation de type I).

Ces dérivés de l'oxygène sont extrêmement réactifs et toxiques et ils oxydent tous les constituants tissulaires situés à leur contact.

De plus, ils diffusent très peu et ont une courte durée de vie de sorte que l'effet phototoxique induit en éclairant l'agent photosensibilisant reste localisé.

Les principales indications de la thérapie photodynamique concernent principalement le domaine de la cancérologie, de la virologie, de l'ophtalmologie, notamment dans le traitement de la dégénérescence maculaire liée à l'age (DMLA), de la cardiologie (resténose après angioplastie), de la rhumatologie et de la dermatologie.

Un grand nombre de molécules photosensibilisantes ont été étudiées pour leur utilisation dans la thérapie photodynamique.

L'utilisation de dérivés d'hématoporphyrine et notamment du Photofrin ® (le porfimère sodique, un mélange d'éthers et d'esters de dihématoporphyrine commercialisé par Quadra Logic Technologies, Inc. Vancouver, BC, Canada) dans la thérapie photodynamique a été largement étudiée (voir par exemple Dougherty, T. J., Photochem. Photobiol., 46, 569 (1987); Kessel, et al., Photochem. Photobiol., 46 563 (1987); Dougherty, T. J., Semin. Surg. Oncol., 2, 24 (1986); McCaughan, J. S., Photochem. Photobiol. 46, 903 (1987); et Gomer, C. J., Semin. Hematol., 26(1) 27 (1989)).

Dans la plupart des études cliniques, il a été utilisé un dérivé d'hématoporphyrine (HPD) qui était constitué d'un mélange de composés de porphyrine (monomères de porphyrine, d'hématoporphyrine, d'hydroxy-éthyl-vinyl-deutéroporphyrine, de protoporphyrine et de dimères/polymères de porphyrine) (voir par exemple, Dougherty, T. J., Photochem. Photobiol. 46, 569 (1987).

Suite à ces études, il s'est révélé que l'utilisation thérapeutique de ces dérivés de l'hématoporphyrine présentait deux inconvénients majeurs.

Le premier est que, à la longueur d'onde de la lumière qui active le composé, la lumière ne pénètre pas assez profondément dans le tissu (voir par exemple Doiron, et al., pp. 281-291 dans : Porphyrins in Tumor Phototherapy, Edited by A. Andreoni and R. Cuulbreddu, Plenum Press, New York, 1984) de sorte que l'utilisation de ces composés est limitée au traitement de tumeurs de taille réduite et de plus ne permet pas de traiter des tumeurs localisées en profondeur dans un tissu.

Le second inconvénient majeur de ces dérivés de l'hématoporphyrine est qu'ils ont tendance à s'accumuler dans les tissus du patient, provoquant notamment une photosensibilisation cutanée et oculaire qui peut se prolonger pendant quatre à huit semaines après le traitement, ce qui nécessitera de prendre des précautions particulières comme par exemple ne pas s'exposer au soleil, mettre une crème à écran total, et porter des vêtements adaptés et des lunettes de soleil.

A titre de seul exemple de ces dérivés d'hématoporphyrine ayant obtenu des autorisations de mise sur le marché (AMM) pour son utilisation dans le traitement par thérapie photodynamique on peut citer le Photofrin ® (par exemple octroi d'AMM aux USA en 1994 et en Europe dès 1996), une solution aqueuse injectable contenant un mélange de composés de porphyrine absorbant la lumière à 630 nm et provoquant une photosensibilisation cutanée et oculaire se poursuivant pendant encore deux mois après le traitement.

Afin de palier aux inconvénients des dérivés d'hématoporphyrine indiqués ci-dessus, il est apparu une nouvelle génération d'agents photosensibilisants ayant comme caractéristique commune que ce sont des composés purs ayant une structure bien définie et qu'ils absorbent la lumière à une longueur d'onde se situant dans la gamme de 650 à 800 nm.

Comme exemples d'agents photosensibilisants utilisés en thérapie photodynamique et appartenant à cette seconde génération, on peut citer les dérivés de la benzoporphyrine (voir par exemple : A.M. Richter, S. Yip, H. Meadows, et al. "Photosensitizing potencies of the structural analogues of benzoporphyrin derivative in different biological test systems" J Clin Laser Med Surg 1996, 14, 335-341; J.G. Levy, A. Chan, H.A. Strong "The clinical status of benzoporphyrin derivative" SPIE 1996, 2625 : 86-95; U. Schmidt-Erfurht, W. Bauman, E. Gragoudas, et al. "Photodynamic therapy of experimental choroidal melanoma using lipoprotein-delivered benzoporphyrin" Ophtalmology 1994, 101 : 89-99).

Les dérivés de la benzoporphyrine ont comme avantage que le composé est activé par une lumière ayant une longueur d'onde plus élevée (- 690 nm) que celle qui active les dérivés de l'hématoporphyrine de sorte que la lumière pénètre plus profondément dans les tissus.

De plus, l'utilisation de ces dérivés de la benzoporphyrine s'est révélée diminuer considérablement l'effet de photosensibilisation cutanée et oculaire.

Cependant, les dérivés de la benzoporphyrine (BPD) ont comme inconvénient majeur d'être insolubles en milieu aqueux de sorte que leur application pharmaceutique par voie parentérale se révèle difficile.

Le brevet US-A-5.289.378 propose de pallier à cet inconvénient en incorporant les dérivés de la benzoporphyrine dans des liposomes.

Comme exemple de formulation injectable liposomale contenant un dérivé de la benzoporphyrine utilisée en thérapie photodynamique pour traiter des formes néovasculaires centrales à prédominance visible de la dégénérescence maculaire liée à l'age (DMLA), on peut citer la Visudyne® (Ciba-Vision) qui contient, en tant que molécule photosensibilisante, le verteporfin absorbant la lumière à une longueur d'onde de 690 nm et provoquant une photosensibilisation cutanée et oculaire limitée dans le temps.

Comme autres exemples d'agents photosensibilisants utiles en thérapie photodynamique appartenant à cette nouvelle génération, on peut notamment citer les dérivés de bactériochlorophylle de formule générale suivante : 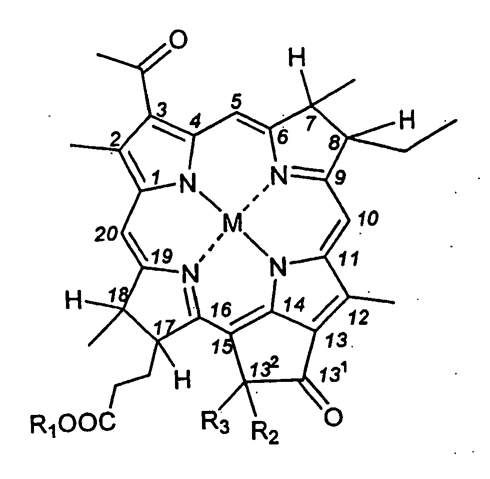 dans laquelle M représente un atome de métal ou 2 atomes d'hydrogène et R₁, R₂ et R₃ représentent chacun indépendamment un atome d'hydrogène ou un groupe variable.

Ces composés sont par exemple dénommés :
**Bchl** ou bactériochlorophylle lorsque :
   M représente Mg
   R₁ représente un groupe phytyle
   R₂ représente un atome H
   R₃ représente un groupe -COOCH₃;
**Bchlide** ou bactériochlorophyllide lorsque :
   M représente Mg
   R₁ représente un atome H
   R₂ représente un atome H
   R₃ représente un groupe -COOCH₃; et
**BPhéide** ou bactériophéophorbide lorsque:
   M représente 2 atomes H et
   R₁ représente un atome H
   R₂ représente un atome H
   R₃ représente un groupe -COOCH₃.

Certains de ces dérivés sont notamment décrits dans DE-A-41 21 876, EP-A-0584 552 et WO 97/19081.

D'autres dérivés de bactériochlorophylle qui se sont révélés particulièrement intéressants sont les dérivés de bactériochlorophylle dans lesquels l'atome de magnésium a été remplacé par un atome de palladium comme ceux décrits par exemple dans WO 00/33833.

Parmi ces dérivés de bactériochlorophylle modifiés au niveau du métal et décrits dans WO 00133833**,** les composés ayant la formule générale (I) suivante : 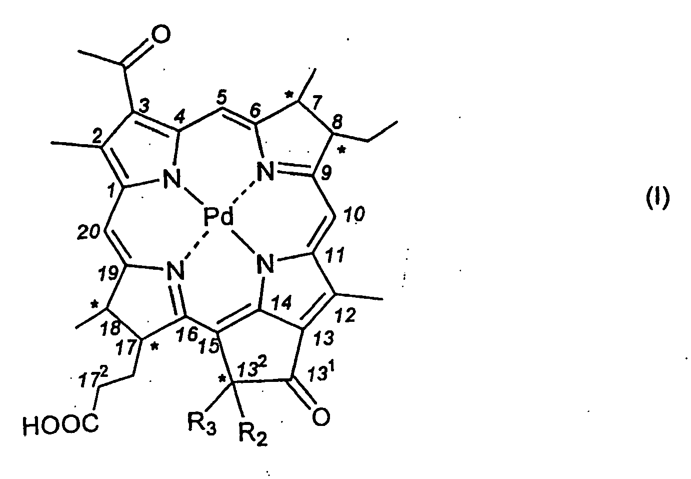 dans laquelle
- R₂: représente un groupe H, OH ou COOR₄, où R₄ est un hydrogène ou un alkyle en C₁-C₁₂ ou un cycloalkyle en C₃-C₁₂,
- R₃: représente H, OH, ou un alkyle ou alcoxy en C₁-C₁₂ et
- *: représente un carbone asymétrique,
ou les sels de ceux-ci, se sont révélés particulièrement intéressants, et parmi ces composés de Formule (I) ci-dessus, le Pd-bactériophéophorbide a ayant la Formule (II) suivante : 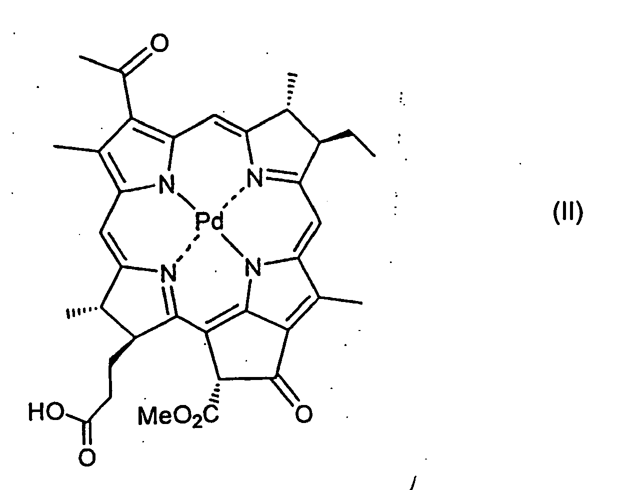 ou un sel de celui-ci, s'est révélé avoir une phototoxicité et une stabilité dans le temps très supérieures aux autres dérivés de bactériochlorophylle.

De plus, le Pd-bactériophéophorbide a, également dénommé Pd-Bphéide a, absorbe la lumière à une longueur d'onde d'environ 760 nm, c'est-à-dire à une longueur d'onde plus élevée que les autres photosensibilisants, ce qui permet une pénétration plus profonde de la lumière dans les tissus, et il ne provoque peu ou pas de photosensibilisation cutanée.

Cependant, tous les composés ayant la Formule (I) ci-dessus ont un caractère lipophile très marqué avec une forte tendance à s'agréger en milieu aqueux ce qui rend leur application pharmaceutique par voie parentérale difficile.

WO 00/33833 revendique notamment une composition pharmaceutique comprenant un composé de Formule (I) ci-dessus, de préférence le Pd-Bphéide a de Formule (II) ci-dessus, et un support pharmaceutiquement acceptable.

Dans la description de WO 00/33833**,** il est toutefois mentionné que pour préparer une telle composition pharmaceutique administrable par voie systémique, il est nécessaire d'ajouter une quantité d'agent tensioactif non-toxique et suffisante pour solubiliser le composé.

Les compositions contenant le Pd-Bphéide a décrites spécifiquement dans WO 00/33833 et utilisées pour les tests *in vivo* chez l'animal contiennent une très grande quantité de Crémophore ® EL (20 - 50%) qui s'est révélée nécessaire pour solubiliser l'agent photosensibilisant.

Dans ces compositions, le Crémophore ® EL joue le rôle d'agent tensioactif permettant de diminuer la formation d'agrégat et de solubiliser le Pd-Bphéide a.

Le Crémophore ® EL est un excipient émulsifiant de type huile de ricin polyoxyéthylénée disponible dans le commerce qui est connu pour son utilisation dans des compositions pharmaceutiques en raison de sa capacité à solubiliser une grande variété de principes actifs lipophiles.

Cependant, une composition contenant plus de 5 % de Crémophore ® EL est difficilement utilisable par voie systémique chez l'homme en raison du risque connu de développer des effets indésirables de type allergique.

Des essais visant à remplacer le Crémophore ® EL par d'autres agents tensioactifs connus pour leur utilisation comme agents de solubilisation de principes lipophiles dans des solutions injectables, comme le Solutol HS 15 ™, le propylène glycol, le polysorbate 80, les lécithines de soja, les lécithines d'oeuf et la N-méthyl-pyrrolidone (Pharmasolve ®), n'ont pas conduit à des résultats satisfaisants.

En raison des propriétés particulièrement intéressantes des composés de Formule (I) ci-dessus, et en particulier du Pd-Bphéide a de Formule (II) ci-dessus, comme agents photosensibilisants en thérapie photodynamique, il est nécessaire de fournir une formulation galénique contenant un composé de Formule (I) ci-dessus qui peut être administrée par voie intraveineuse chez l'homme sans risque de complications dues à un excipient.

Le but de la présente invention est de fournir une formulation galénique injectable dans laquelle le composé de Formule (I) ci-dessus est complètement solubilisé et qui contient une quantité fortement réduite de tensioactif.

Ce but a été atteint après que les inventeurs aient constaté avec surprise que l'ajout d'une petite quantité de cosolvants non-aqueux, à savoir l'ajout d'une petite quantité d'un mélange d'alcool benzylique/éthanol ou de propylène glycol au Pd-Bphéide a, permettait de solubiliser le Pd-Bphéide a dans un milieu aqueux après sa salification.

Selon un premier aspect, la présente invention concerne une formulation galénique injectable pour utilisation dans un diagnostic ou une thérapie photodynamique (PDT), ladite formulation galénique contenant :
- un composé représenté par la formule générale (I) suivante : 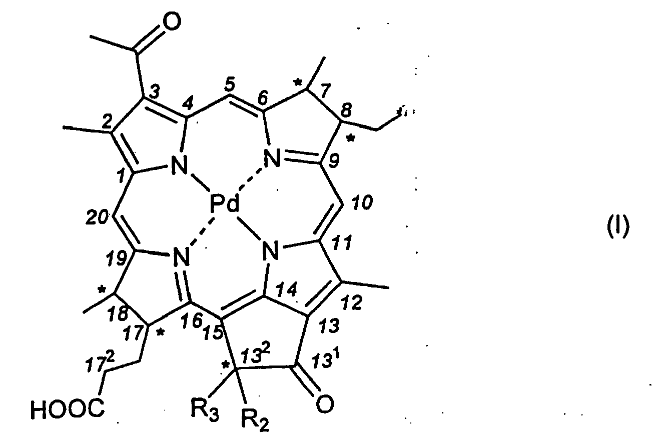 dans laquelle
   - R₂: représente un groupe H, OH ou COOR₄, où R₄ est un hydrogène ou un alkyle en C₁-C₁₂ ou un cycloalkyle en C₃-C₁₂,
   - R₃: représente H, OH, ou un alkyle ou alcoxy en C₁-C₁₂ et
   - *: représente un carbone asymétrique
   sous la forme d'un sel de métal alcalin, dans une quantité ne dépassant pas 10 mg/ml, en tant qu'agent photosensibilisant, et
- un véhicule en phase aqueuse pharmaceutiquement acceptable,
caractérisée en ce que le véhicule en phase aqueuse pharmaceutiquement acceptable contient au moins un mélange alcool benzylique - éthanol ayant un rapport de mélange de 15 : 85 à 25 : 75 ou du propylène glycol en tant qu'agent de solubilisation du photosensibilisant et un tensioactif permettant de diminuer l'agrégation du photosensibilisant dans une quantité ne dépassant pas 20 % en poids par rapport au poids total de la formulation. avec la condition que si le tensioactif est un crémophore sa quantité ne doit pas dépasser 5% en poids par rapport au poids total.

Selon un second aspect, la présente invention concerne un procédé de préparation d'une formulation galénique injectable pour utilisation dans un diagnostic ou une thérapie photodynamique (PDT) contenant un composé représenté par la Formule (I) suivante : 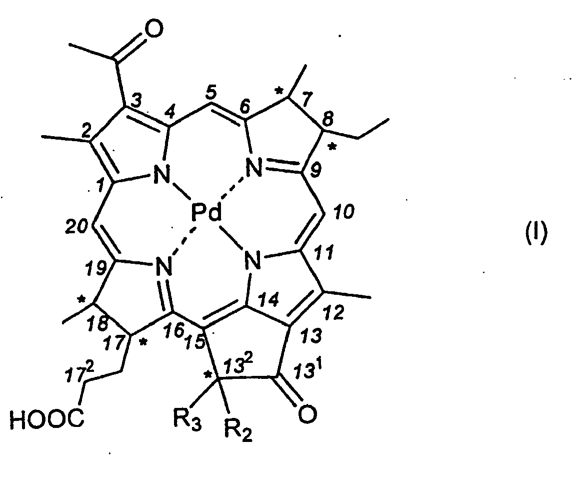 dans laquelle
- R₂: représente un groupe H, OH ou COOR₄, où R₄ est un hydrogène ou un alkyle en C₁-C₁₂ ou un cycloalkyle en C₃-C₁₂,
- R₃: représente H, OH, ou un alkyle ou alcoxy en C₁-C₁₂ et
- *: représente un carbone asymétrique
sous la forme de sel de métal alcalin, dans une quantité allant jusqu'à 10 mg/ml en tant qu'agent photosensibilisant et un véhicule en phase aqueuse pharmaceutiquement acceptable, ledit procédé comprenant, dans l'ordre indiqué, les étapes consistant à :
(1) mouiller l'agent photosensibilisant de Formule (I) sous sa forme acide avec un agent de solubilisation constitué d'un mélange alcool benzylique - éthanol dans un rapport de mélange de 15 : 85 à 25 : 75 ou de propylène glycol;
(2) ajouter un agent tensioactif dans une quantité ne dépassant pas 20 % en poids par rapport au poids final de la formulation;
(3) ajouter un hydroxyde de métal alcalin dans une quantité suffisante pour neutraliser la fonction acide de l'agent photosensibilisant de Formule (I);
(4) agiter le mélange en phase aqueuse de manière suffisante pour permettre la salification complète de l'agent photosensibilisant et sa totale solubilisation;
(5) ajouter un agent tampon destiné à ajuster le pH à une valeur physiologiquement acceptable de 7,2 ± 0,4;
(6) si nécessaire, ajuster le volume de la formulation avec de l'eau pour préparation injectable.

Selon un troisième aspect, la présente invention concerne une formulation galénique injectable obtenue par ce procédé.

La formulation galénique de la présente invention contient une quantité fortement réduite de tensioactif dont le rôle essentiel est de diminuer la formation d'agrégat.

La présente invention fournit ainsi une formulation galénique dans laquelle l'agent photosensibilisant, à savoir un composé de Formule (I) ci-dessus sous forme de sel, est complètement solubilisé, et qui peut être injectée à moindre risque à l'homme dans le cadre d'une thérapie photodynamique.

D'autres avantages de la présente invention apparaîtront à la lecture de la description détaillée de l'invention qui suit.

### DESCRIPTION DETAILLEE DE L'INVENTION

Conformément à la présente invention, la formulation galénique injectable pour utilisation dans une thérapie photodynamique contient
- un composé de Formule (I) 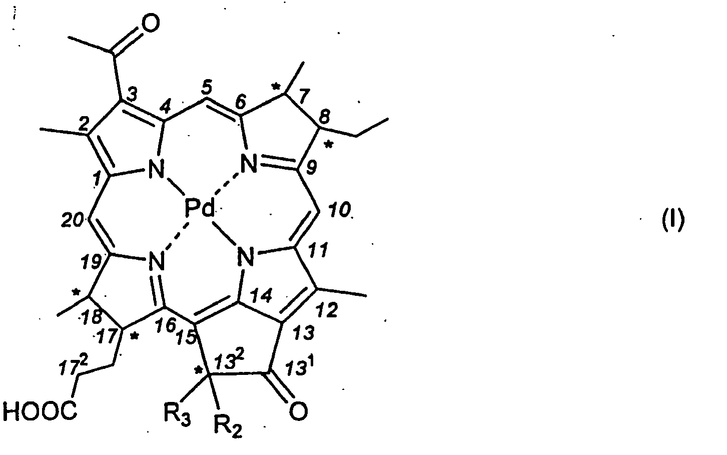 dans laquelle
   - R₂: représente un groupe H, OH ou COOR₄, où R₄ est un hydrogène ou un alkyle en C₁-C₁₂ ou un cycloalkyle en C₃-C₁₂,
   - R₃: représente H, OH, ou un alkyle ou alcoxy en C₁-C₁₂ et
   - *: représente un carbone asymétrique,
   sous la forme d'un sel de métal alcalin, en tant qu'agent photosensibilisant, et
   - un véhicule en phase aqueuse pharmaceutiquement acceptable.

Dans la formulation galénique de la présente invention, le composé de Formule (1) ci-dessus est de préférence le Pd-Bphéide a de Formule (II) ci-dessous : 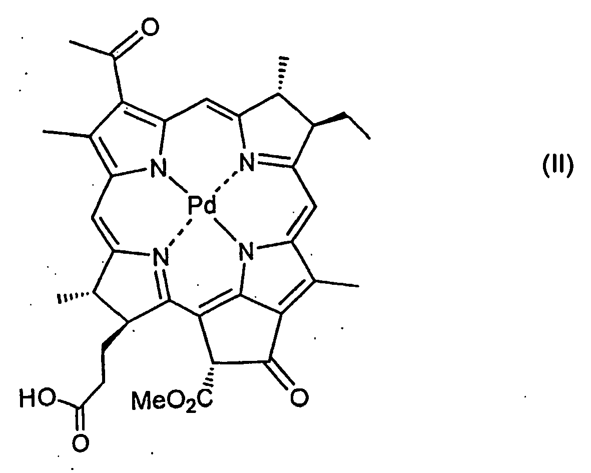 sous la forme d'un sel de métal alcalin.

Le sel de métal alcalin du composé de Formule (I) ou du composé de Formule (II) peut être un sel de sodium ou un sel de potassium.

De préférence, le sel de métal alcalin du composé de Formule (I) ou du composé de Formule (II) est un sel de sodium.

Les composés de Formule (I) ci-dessus, et en particulier le Pd-Bphéide a représenté par la Formule (II) ci-dessus, ainsi que leurs sels de métal alcalin, peuvent être préparés comme décrit dans WO 00/33833**.**

La quantité du composé photosensibilisant contenue dans la formulation galénique injectable de la présente invention ne doit pas être supérieure à 10 mg/ml, car à une quantité supérieure à 10 mg/ml, le composé photosensibilisant ne serait pas complètement solubilisé.

Lorsque la formulation galénique injectable est destinée à être utilisée dans le cadre d'une thérapie photodynamique, la quantité d'agent photosensibilisant contenue dans la formulation ne devrait de préférence pas être inférieure à 0,1 mg/ml, car au-dessous de cette concentration, le volume de formulation à administrer serait trop important.

Toutefois, des quantités d'agent photosensibilisant inférieures à 0,1 mg/ml peuvent être envisagées lorsque la formulation galénique injectable de la présente invention est destinée à être utilisée dans le cadre d'un diagnostic photodynamique.

Le véhicule en phase aqueuse pharmaceutiquement acceptable contenu dans la formulation galénique injectable de la présente invention a pour but de solubiliser l'agent photosensibilisant tout en fournissant une stabilité dans le temps à la formulation.

Ce véhicule en phase aqueuse pharmaceutiquement acceptable contient au moins un mélange alcool benzylique-éthanol ou du propylène glycol en tant qu'agent de solubilisation de l'agent photosensibilisant et un agent tensioactif permettant de diminuer l'agrégation de l'agent photosensibilisant dans une quantité ne dépassant pas 20 % en poids par rapport au poids total de la formulation.

Lorsque le véhicule pharmaceutique contient un mélange alcool benzylique-éthanol en tant qu'agent de solubilisation, le rapport de mélange alcool benzylique : éthanol doit être de 15 : 85 à 25 : 75 v/v.

Dans le cas d'un rapport de mélange inférieur à 15 en alcool benzylique, l'agent photosensibilisant n'est pas complètement solubilisé.

Dans le cas d'un rapport de mélange supérieur à 25 en alcool benzylique, le mélange n'est plus miscible à l'eau.

Dans une forme d'exécution particulièrement préférée de l'invention, le véhicule contient un mélange alcool benzylique-éthanol dans un rapport de mélange alcool benzylique : éthanol de 20 : 80.

Le mélange alcool benzylique - éthanol est de préférence contenu dans le véhicule dans une quantité ne dépassant pas 5 % en volume par rapport au volume total de la formulation.

L'alcool benzylique et l'éthanol contenus dans la formulation galénique injectable de la présente invention doivent répondre aux exigences de pureté requises pour leur utilisation dans des formulations injectables, conformément à la

Pharmacopée Européenne et à la Pharmacopée américaine (USP).

Lorsque le véhicule pharmaceutique contient du propylène glycol en tant qu'agent mouillant, le propylène glycol est de préférence contenu dans le véhicule dans une quantité ne dépassant pas 5 % en volume par rapport au volume total de la formulation.

Le propylène glycol contenu dans la formulation galénique injectable de la présente invention doit répondre aux exigences de pureté requises pour son utilisation dans des formulations injectables, conformément à la Pharmacopée Européenne et à la Pharmacopée américaine (USP).

L'agent tensioactif contenu dans le véhicule en phase aqueuse pharmaceutiquement acceptable peut être n'importe quel agent tensioactif ayant le capacité de diminuer l'agrégation de l'agent photosensibilisant dans la formulation galénique injectable et il doit être contenu dans une quantité ne dépassant pas 20 % en poids par rapport au poids total de la formulation.

Cet agent tensioactif peut être par exemple, le Crémophore ® EL (BASF, Allemagne), le Crémophore ® EL P (BASF, Allemagne), le Solutol HS 15™ (BASF, Allemagne), le propylène glycol, le polysorbate 80, les lécithines de soja, les lécithines d'oeuf et la N-méthyl-pyrrolidone (Pharmasolve ®, BASF, Allemagne).

De préférence, l'agent tensioactif est le Crémophore ® EL, le Crémophore ® EL P ou le Solutol HS 15 ™.

Une formulation particulièrement préférée de la présente invention contient, en tant qu'agent tensioactif, du Crémophore ® EL ou du Crémophore ® EL P dans une quantité ne dépassant pas 5 % en volume par rapport au volume total de la formulation.

Dans une forme d'exécution particulièrement préférée de la présente invention, la formulation contient 0,25 % p/p de Pd-Bphéide a, 5 % v/p d'un mélange alcool benzylique-éthanol ayant un rapport de mélange alcool benzylique : éthanol de 20 : 80, et 5 % p/p de Crémophore ® EL P.

La formulation galénique injectable de la présente invention peut être préparée selon un procédé comprenant, dans l'ordre indiqué, les étapes consistant à :
(1) mouiller l'agent photosensibilisant de Formule (I) sous sa forme acide ou de Formule (II) sous sa forme acide avec un agent de solubilisation constitué d'un mélange alcool benzylique - éthanol dans un rapport de mélange de 15 : 85 à 25 : 75 ou de propylène glycol;
(2) ajouter un agent tensioactif dans une quantité ne dépassant pas 20 % en poids par rapport au poids final de la formulation;
(3) ajouter un hydroxyde de métal alcalin en solution aqueuse dans une quantité suffisante pour neutraliser la fonction acide de l'agent photosensibilisant de Formule (I) ou de Formule (II);
(4) agiter le mélange en phase aqueuse de manière suffisante pour permettre la salification complète de l'agent photosensibilisant et sa totale solubilisation;
(5) ajouter un agent tampon destiné à ajuster le pH à une valeur physiologiquement acceptable de 7,2 ± 0,4;
(6) si nécessaire, ajuster le volume de la formulation avec de l'eau pour préparation injectable.

Dans une forme d'exécution préférée de l'invention, l'agent de solubilisation est de préférence ajouté dans une quantité ne dépassant pas 5 % en volume par rapport au volume total de la formulation.

Dans le procédé selon la présente invention, l'agent de solubilisation ajouté pour mouiller l'agent photosensibilisant est de préférence un mélange alcool benzylique-éthanol ayant un rapport de mélange de 20 : 80.

Le mouillage par l'alcool benzylique pur n'est pas possible en raison de la non-miscibilité de l'alcool benzylique avec la solution de soude diluée.

L'ajout d'éthanol à l'alcool benzylique dans les proportions indiquées permet la miscibilité de la phase organique de mouillage avec la solution de soude diluée.

L'alcool benzylique et l'éthanol ou le propylène glycol ajoutés pour mouiller l'agent de photosensibilisation doivent répondre aux exigences de pureté requises pour leur utilisation dans des formulations injectables, conformément à la

Pharmacopée Européenne et à la Pharmacopée américaine (USP).

L'agent tensioactif ajouté pour diminuer l'agrégation de l'agent photosensibilisant dans la formulation galénique injectable est par exemple le Crémophore® EL, le Crémophore ® EL P, le Solutol HS 15 ™, le propylène glycol, le polysorbate 80, les lécithines de soja, les lécithines d'oeuf et la N-méthyl-pyrrolidone (Pharmasolve ®).

De préférence, l'agent tensioactif ajouté est le Crémophore ® EL, le Crémophore ® EL P ou le Solutol HS 15 ™.

La quantité d'agent tensioactif qui est ajoutée variera en fonction de l'agent tensioactif utilisé et de la concentration finale de l'agent photosensibilisant dans la formulation.

Elle ne devra cependant pas être supérieure à 20 % en poids par rapport au poids final de la formulation.

Dans une forme d'exécution particulièrement préférée du procédé de la présente invention, on ajoute en tant qu'agent tensioactif du Crémophore ® EL ou du Crémophore ® EL P dans une quantité ne dépassant pas 5 % en volume par rapport au volume total de la formulation.

L'hydroxyde de métal alcalin utilisé dans le procédé de la présente invention peut être l'hydroxyde de sodium ou l'hydroxyde de potassium et il est ajouté sous la forme d'une solution aqueuse.

L'hydroxyde de métal alcalin est ajouté au composé de Formule (I) sous sa forme acide ou au composé de Formule (II) sous sa forme acide dans une quantité permettant de salifier totalement la fonction acide du composé de Formule (I) ou du composé de Formule (II).

De préférence, l'hydroxyde de métal alcalin utilisé est l'hydroxyde de sodium, de manière à ce que la formulation galénique injectable obtenue contienne le composé de Formule (1), de préférence le composé de Formule (II), sous la forme du sel de sodium.

Une agitation suffisante devra être assurée pour permettre la salification complète de l'agent photosensibilisant et la totale solubilisation de celui-ci dans le véhicule.

L'agent destiné à ajuster le pH utilisé dans le procédé de la présente invention peut être par exemple l'acide citrique, l'acide phosphorique et ses sels de sodium (monobasique et dibasique), l'acide D-tartrique, l'acide L-tartrique, l'acide D-malique, l'acide L-malique.

L'agent destiné à ajuster le pH est ajouté en quantité nécessaire pour tamponner la formulation injectable à un pH physiologiquement acceptable de 7,2 ± 0,4.

Dans une forme d'exécution particulièrement préférée de la présente invention, l'agent destiné à ajusté le pH ajouté est l'acide citrique car il confère à la formulation une stabilité dans le temps et en présence d'air améliorée.

Afin d'améliorer la stabilité de la formulation injectable obtenue par le procédé de la présente invention, on effectuera de préférence les différentes étapes (1) à (6) du procédé sous atmosphère de gaz inerte.

Dans la formulation galénique injectable obtenue par le procédé de la présente invention, le composé de Formule (I) ou de préférence le composé de Formule (II), est complètement solubilisé, ceci étant montré par la limpidité et l'absence totale de précipité.

De plus, la formulation injectable obtenue par le procédé de la présente invention présente une excellente stabilité dans le temps.

Dans la composition galénique de la présente invention, le composé de Formule (I), et de préférence le composé de Formule (II), est sous forme de monomère avec cependant une certaine quantité du composé sous forme d'agrégat (oligomères).

Cependant, lors de l'injection de la formulation galénique injectable de la présente, la forme oligomérique présente dans la formulation se transforme rapidement en monomère dès qu'il entre en contact avec le liquide physiologique, en particulier le sang, de sorte que le produit photosensibilisant actif est entièrement sous forme de monomère.

L'analyse de la formulation injectable de la présente invention par spectroscopie UV peut fournir des indications intéressantes sur le Pd-Bphéide a contenu dans la formulation puisque le Pd-Bphéide a présente un pic voisin de 760 nm, l'agrégation de celui-ci peut être déterminée par l'apparition d'un pic voisin de 815 nm environ, et la dégradation de celui-ci, par oxydation notamment, peut être déterminée par des déplacements hypsochromes des maxima, notamment celui voisin de 760 nm.

Les exemples suivants sont destinés à illustrer la présente invention. Cependant, ils ne doivent en aucun cas être considérés comme limitant l'étendue de la présente invention.

### EXEMPLES

Dans les exemples ci-dessous, tous les réactifs utilisés sont des réactifs disponibles dans le commerce, provenant notamment de chez SDS (BP 4- Valdonne, F-13124 Peypin), Merck Eurolab (Europarc, F-33608 Pessac Cedex), BASF (Allemagne), Flüka (Buchs, Suisse), etc, à moins qu'autre chose ne soit spécifié.

### PREPARATION DE L'AGENT PHOTOSENSIBILISANT Pd-Bphéide a

Remarque : l'ensemble du procédé est réalisé sous lumière inactinique et sous atmosphère d'azote.

### 1) Extraction de la bactériochlorophylle a (BChla)

### Réactifs utilisés:

| | |
|---|---|
| Cellules ***Rhodovolum Sulfidophilum*** | 11 kg |
| Méthanol [SDS ou Merck, purex pour syntheses, pureté ≥ 99.8%] | 126 l |

### Mode opératoire :

Des cellules ***Rhodovolum Sulfidophilum*** lyophilisées (11 kg) ont été extraites par du méthanol (57 l) à 25°C et le milieu a été filtré. Le résidu de cellules a été repris par 47 l de méthanol et filtré. Après rinçage du résidu par 22 l de méthanol, les filtrats ont été regroupés et concentrés sous pression réduite.

Il a été obtenu ainsi environ 3500 g de produit brut contenant 70 g de BChla (quantité de BChla déterminée par dosage UV).

### 2) Synthèse de la bactériophéophorbide a

### Réactifs utilisés :

| | |
|---|---|
| Produit brut contenant 70 g Bactériochlorophylle a obtenu à l'étape 1) | 3500 g |
| Acide trifluoroacétique [SDS, pur pour synthèses, pureté ≥ 99%] | 7 l |
| Chloroforme [SDS, pur pour synthèses, pureté ≥ 99.9%] | 11,2 l |

### Mode opératoire :

Le produit brut contenant 70 g de Bactériochlorophylle a (3500 g) a été solubilisé dans le chloroforme (11,2 l) et l'acide trifluoroacétique (7 l) a été ajouté. L'avancement de la réaction a été suivi par CCM. Lorsqu'on ne détectait plus de produit de départ, 56 l d'eau et 14 l de chloroforme ont été ajoutés au milieu réactionnel. Après agitation puis décantation, les deux phases ont été séparées. La phase organique a été lavée à l'eau (25 l) et le chloroforme a été concentré sous pression réduite jusqu'à un volume de 6 l.

La Bphéide brute a été obtenue en solution dans le chloroforme et n'a pas été isolée avant purification.

### 3) Purification de la bactériophéophorbide a

### Réactifs :

| | |
|---|---|
| Solution de Bphéide brute dans le chloroforme obtenue à l'étape 2) Cyclohexane [SDS, pur pour synthèses, pureté ≥ 99%] | 101 |
| Pentane [SDS, pur pour synthèses, pureté ≥ 99%] | 71 |
| Méthanol [SDS ou Merck, purex pour synthèses, pureté ≥ 99.8%] | 31 |
| Silice [SDS, 60 A C.C., 70-200 µm] | 300 g |
| Acétate d'éthyle [Merck, pur, pureté 99.5%] | 61 |

### Mode opératoire :

Le cyclohexane (10 l) a été ajouté à la solution de Bphéide brute dans le chloroforme et les solvants ont été distillés sous pression réduite avec ajout de cyclohexane jusqu'à élimination du chloroforme et jusqu'à obtenir un volume de 7 l. Le produit a alors été précipité par ajout de pentane (7 l), agité pendant 15 minutes à 30°C et filtré. Après séchage en étuve à vide (30°C), il a été obtenu 60 g de produit semi-purifié (pureté UV : 50-60 %).

Le produit semi-purifié (60 g) a été dissous dans un mélange chloroforme/méthanol 95/5 (6 l) et 300 g de silice ont été ajoutés. Après 10 minutes d'agitation, le milieu a été filtré. Le filtrat a été dilué avec 6 l d'acétate d'éthyle et les solvants ont été distillés sous pression réduite. Au fur et à mesure de la concentration, de l'acétate d'éthyle a été ajouté jusqu'à obtenir une densité du distillat égale à 0,9. Le volume de la solution a alors été amené à 3.0 l. La solution a été filtrée et la poudre obtenue a été séchée à l'étuve sous vide à 30°C.

Il a été isolé environ 35 g de Bphéide purifiée (pureté UV : 80%).

### 4) Synthèse du palladium-bactériophéophorbide a

### Réactifs :

| | |
|---|---|
| Bphéide purifiée obtenue à l'étape 3) | 6,25 g |
| Acétate de palladium [SDS ou MERCK, teneur ≥ 99% w/w] | 13,15 g |
| Acide ascorbique [SDS, pureté ≥ 99%] | 105 g |
| chloroforme [SDS, pur pour synthèses, pureté ≥ 99.9%] | 2,2 l |
| Méthanol [SDS, purex pour analyses, pureté ≥ 99.8%] | 3,3 l |
| Sulfate de sodium anhydre [SDS, pour analyses, pureté ≥ 99.5%] | 1,5 Kg |

### Mode opératoire :

L'acide ascorbique (105 g) a été dissous dans 3,3 l de méthanol et le milieu a été chauffé à 35°C. La Bphéide (6,25 g) solubilisée dans 1,5 l de chloroforme a été additionnée en 10 minutes au milieu précédent. Après 10 minutes d'agitation, l'acétate de palladium (13,15 g) solubilisé dans 0,7 l de chloroforme a été additionné en 10 minutes au milieu réactionnel. L'avancement de la réaction a été suivi par UV. Après 15 minutes (insertion totale) le mélange a été ramené à 20°C puis il a été versé sur 20 l d'eau. Après agitation et décantation, les phases ont été séparées. La phase organique a été lavée à l'eau (20 l) jusqu'à pH ≥ 5 puis séchée sur sulfate de sodium (1,5 kg). Après concentration des solvants sous pression réduite, il a été obtenu 5,85 g de produit brut (pureté UV : 85%).

### 5) Purification du palladium-bactériophéophorbide a

### Réactifs utilisés:

| | |
|---|---|
| Pd-Bphéide brut obtenue à l'étape 4) | 5.85 g (pureté UV : 85%) |
| Chloroforme [SDS, pur pour synthèses, pureté ≥ 99.9%] | 175 ml |

### Mode opératoire :

Le produit brut (5,85 g) a été repris dans le chloroforme (175 ml) et agité pendant 5 minutes. La suspension a été filtrée et le solide obtenu a été séché à l'étuve (30°C) sous vide.

Il a été isolé 5,0 g de Pd-Bphéide a purifié (pureté HPLC > 97 %).

UV/VIS dans le chloroforme : λₘₐₓ[nm] (intensité relative 330 (0,798); 385 (0,597); 535 (0,292); 760 (1,711).
¹H-RMN dans l'acétone-D₆ [ppm] : 9,27, 8,86, 8,77 (chaque s, 1H, 5-, 10-, 20-H); 6,01 (s, 1H, 13²-H); 4,7 à 4,0 (4 m, 1 H, 7-, 8-, 17-, 18-H); 3,86 (s, 3H, 13²-CO₂CH₃); 3,51 (s, 3H, 2-CH₃); 3,34 (s, 3H, 12-CH₃); 3,07 (s, 3H, 3-COCH₃); 2,7 à 2.1 (3m, 2H, 8-, 17¹-, 17 ²-CH₂); 1,79, 1.69 (chaque d, 3H, 7-, 18-CH₃); 1,09 (t, 3H, 8-CH₂CH₃).

SM calculé pour C₃₅H₃₆N₄O₆Pd: 714,1670 (M⁺). Trouvé : 714,1688 (M⁺).

| Analyse élémentaire : | | | | | |
|---|---|---|---|---|---|
| Calculé (%) : | C : 58,79 | H : 5,07 | N : 7,83 | O : 13,42 | Pd : 14,88 |
| Trouvé (%) : | C : 59,13 | H : 5,12 | N : 7,72 | O : 13,33 | Pd : 14,69 |

Le Pd-Bphéide a obtenu ci-dessus a été utilisé pour préparer les formulations injectables ci-dessous.

### Formulation injectable 1

Formulation 1 contenant 2,5 mg/ml de Pd-Bphéide a comme agent photosensibilisant

### Véhicule :

Phase organique : alcool benzylique/éthanol (20/80) 5 % v/p
Tensioactif : Crémophore® EL P 5 % p/p
NaOH : 0,028 M
pH ajusté à 7,2 ± 0,4 par l'acide citrique 0,01 M

La formulation 1 a été préparée comme suit :

Dans un récipient en verre de dimension appropriée contenant 2,5 mg de Pd-Bphéide a préparé comme décrit ci-dessus, il a été ajouté 50 µl d'un mélange 20:80 v/v d'alcool benzylique et d'éthanol et le mélange a été légèrement agité.

Ensuite, il a été ajouté au mélange 50 mg de Crémophore ®EL P et le mélange a été agité au vortex. Ensuite, il a été ajouté au mélange 500 µl d'une solution d'hydroxyde de sodium NaOH à 8,4 x 10⁻³ M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la Formulation 1 est représentée sur le Tableau 1 suivant :

**Tableau 1**

| Nom des composants | Formule | | Fonctions | Référence aux normes |
|---|---|---|---|---|
| | unitaire | centésimale (%) | | |
| ***Agent photosensibilisant*** | | | | |
| Pd-Bphéide a | 2,5 mg | 0,25 % p/p | PA | |

| ***Autres composants*** | | | | |
|---|---|---|---|---|
| Ethanol absolu | 40 µl | 4 % v/p | Co-solvant | PE 4^{ème} édition 1318, A2000 |
| Alcool benzylique | 10 µl | 1 % v/p | Co-solvant | PE 4^{ème} édition 0256, 1997 |
| Crémophore®EL P | 50 mg | 5 % p/p | Tensioactif | PE 4^{ème} édition 1082, 1997 |
| NaOH 8,4 x 10⁻³ M | 500 µl | 50 % v/p | Agent salifiant | PE 4^{ème} édition 0677, 1997 |
| Acide citrique 0,01 M | QS 7,2 ± 0,4 | QS 7,2 ± 0,4 | Agent tampon | PE 4^{ème} édition 0456, 1997 |
| EPPI QS | 1 g | 100 | Diluant | PE 4^{ème} édition 0169,A2000 |

Cette Formulation 1 de la présente invention est limpide, fortement colorée (pourpre foncé) et elle ne contient aucune trace de précipité, ce qui montre que le Pd-Bphéide a est complètement solubilisé.

Le spectre d'absorption UV du Pd-Bphéide a dans cette Formulation 1 de la présente invention montrait 5 pics principaux à 344, 385, 538, 763 et 818 nm avec un rapport dé DO 763/818 supérieur à 1, indiquant que le monomère représente au niveau spectral plus de 50 % du produit.

Selon les conditions ICH, cette Formulation 1 est stable à 4°C pendant au moins 6 mois, ceci étant vérifié par dosage HPLC et UV.

Des études de toxicité de la Formulation injectable 1 ont été réalisées dans différentes espèces animales après administration intraveineuse unique ou répétée :
- Chez le rat mâle (Sprague Dawley) chez 6 à 12 animaux par dose (2 x 5, 2 x 10 et 2 x 20 mg/kg). Le produit a été injecté par voie intraveineuse dans la veine de la queue.
- Chez le lapin (Albino de Nouvelle-Zélande) chez 5 animaux à la dose de 5 mg/kg. Le produit a été injecté 3 fois par voie intraveineuse, 1 fois par voie intra-artérielle et 1 fois par voie périveineuse.
- Chez le singe (Cynomolgus d'un poids variant de 2,5 à 3,2 kg) chez 6 à 10 animaux par sexe et par dose (1, 2,5 et 6 mg/kg). Le produit a été injecté par voie intraveineuse dans la veine saphène ou céphalique une fois par jour pendant 14 jours.
- Chez le singe (Cynomolgus d'un poids variant de 2,25 à 4,7 kg) chez 3 à 6 animaux par dose (2 et 5 mg/kg). Le produit a été injecté par voie intraveineuse dans la veine saphène ou céphalique.

L'évaluation des animaux a été réalisée par l'analyse des signes cliniques, des examens biologiques au niveau du sang et des urines, des examens cardiovasculaires et des examens histopathologiques.

Ces études ont montré que le Pd-Bphéide a et sa Formulation injectable 1 étaient bien tolérés jusqu'à des vitesses d'injection pouvant aller jusqu'à 4 ml/min.

### Formulation injectable 2

Formulation 2 contenant 5 mg/ml de Pd-Bphéide a comme agent photosensibilisant

### Véhicule :

Phase organique : alcool benzylique/éthanol (20/80) 5 % v/p
Tensioactif : Crémophore ® EL 5 % p/p
NaOH : 0,028 M
pH ajusté à 7,2 ± 0,4 par l'acide citrique 0,01 M

La formulation 2 a été préparée comme suit :

Dans un récipient en verre de dimension appropriée contenant 5 mg de

Pd-Bphéide a préparé comme décrit ci-dessus, il a été ajouté 50 µl d'un mélange 20:80 v/v d'alcool benzylique et d'éthanol et le mélange a été légèrement agité.

Ensuite, il a été ajouté au mélange 50 mg de Crémophore ®EL et le mélange a été agité au vortex. Ensuite, il a été ajouté au mélange 500 µl d'une solution d'hydroxyde de sodium NaOH à 0,028 M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la Formulation 2 est représentée sur le Tableau 2 suivant :

**Tableau 2**

| Nom des composants | Formule | | Fonctions | Référence aux normes |
|---|---|---|---|---|
| | unitaire | centésimale (%) | | |
| ***Agent photosensibilisant*** | | | | |
| Pd-Bphéide a | 5 mg | 0,5 % p/p | PA | |

| ***Autres composants*** | | | | |
|---|---|---|---|---|
| Ethanol absolu | 40 µl | 4 % v/p | Co-solvant | PE 4^{ème} édition 1318, A2000 |
| Alcool benzylique | 10 µl | 1 % v/p | Co-solvant | PE 4^{ème} édition 0256, 1997 |
| Crémophore®EL | 50 mg | 5 % p/p | Tensioactif | PE 4^{ème} édition 1082, 1997 |
| NaOH 0,028 M | 500 µl | 50 % v/p | Agent salifiant | PE 4^{ème} édition 0677, 1997 |
| Acide citrique 0,01 M | QS 7,2 ± 0,4 | QS 7,2 ± 0,4 | Agent tampon | PE 4^{ème} édition 0456, 1997 |
| EPPI QS | 1g | 100 | Diluant | PE 4^{ème} édition 0169,A2000 |

Cette Formulation 2 de la présente invention est limpide, fortement colorée (pourpre foncé) et elle ne contient aucune trace de précipité, ce qui montre que le Pd-Bphéide a est complètement solubilisé.

Le spectre d'absorption UV du Pd-Bphéide a dans cette Formulation 2 de la présente invention montrait 5 pics principaux à 344, 385, 538, 763 et 818 nm avec un rapport de DO 763/818 voisin de 1, indiquant que le monomère représente au niveau spectral 50 % du produit.

Selon les conditions ICH, cette Formulation 2 est stable à 4°C pendant au moins 6 mois, ceci étant vérifié par dosage HPLC et UV.

Les tests biologiques suivants ont permis de montrer le rôle important et la nécessité d'une bonne solubilisation du principe actif ainsi que de la réduction de la forme agrégat par rapport à la forme monomère.

Un test visant à étudier la désagrégation du produit formulé une fois injecté a été réalisé chez le lapin blanc de Nouvelle Zélande d'un poids de 3 à 4 kg.

Trois lapins ont été utilisés pour cette étude.

Les lapins ont reçu une dose de 5 mg/Kg dans la veine de l'oreille soit 3 ml de la Formulation injectable 1 à 5 mg/ml pour un lapin de 3 Kg.

La vitesse d'infusion variait de 0.3 à 1 ml/min.

Le spectre d'absorption du Pd-Bphéide a a été mesuré en continu par réflectance spectroscopique en utilisant un système de détection multi-canaux. Ce système permet la détection du produit au niveau tissulaire sur l'animal vivant. Ceci permet de suivre ainsi l'évolution des agrégats dans l'organisme.

Dans ces conditions, la désagrégation in vivo de l'oligomère en forme monomérique s'opère très rapidement entre 15 et 30 minutes.

L'absence de Crémophore dans la formulation ne permettait pas d'obtenir cette désagrégation in vivo et la concentration de monomère alors produite restait très faible.

Des études de toxicité de la Formulation injectable 2 ont été réalisées dans différentes espèces animales après administration intraveineuse unique ou répétée :
1- Chez la souris (OF, d'un poids variant de 25 à 35 grammes) chez 10 animaux par sexe et par dose (0,2x25, 2x 50, 2x75 et 1x100 mg/kg). Le produit a été injecté dans la veine de la queue.
2- Chez le rat (Sprague Dawley d'un poids variant de 150 à 230 grammes) chez 4 à 10 animaux par sexe et par dose (de 4 à 200 mg/kg). Le produit a été injecté dans la veine de la queue.
3- Chez le singe (Cynomolgus d'un poids variant de 1,8 à 3,5 Kg) chez 1 animal par sexe et par dose (8, 16, et 24 mg/kg). Le produit a été injecté dans la veine saphène ou céphalique une fois par jour pendant 7 jours.

L'évaluation des animaux s'est faite par l'analyse des signes cliniques, des examens biologiques au niveau du sang et des urines, des examens histopathologiques.

Ces études ont montré que le Pd-Bphéide a et sa Formulation injectable 2 étaient bien tolérés à condition de faire des injections lentes pour que les oligomères présents soient progressivement désagrégés en forme monomérique.

### Formulation injectable 3

Formulation 3 contenant 5 mg/ml de Pd-Bphéide a comme agent photosensibilisant

### Véhicule :

Phase organique : alcool benzylique/éthanol (20/80) 5 % v/p
Tensioactif : Solutol HS 15 10 % p/p
NaOH : 0,028 M
pH ajusté à 7,2 ± 0,4 par l'acide citrique 0,01 M

La formulation 3 a été préparée comme suit :

Dans un récipient en verre de dimension appropriée contenant 5 mg de Pd-Bphéide a préparé comme décrit ci-dessus, il a été ajouté 50 µl d'un mélange 20:80 v/v d'alcool benzylique et d'éthanol et le mélange a été légèrement agité. Ensuite, il a été ajouté au mélange 100 mg de Solutol HS 15 10 % p/p et le mélange a été agité au vortex. Ensuite, il a été ajouté au mélange 500 µl d'une solution d'hydroxyde de sodium NaOH à 0,028 M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la Formulation 3 est représentée sur le Tableau 3 suivant :

**Tableau 3**

| Nom des composants | Formule | | Fonctions | Référence aux normes |
|---|---|---|---|---|
| | unitaire | centésimale (%) | | |
| ***Agent photosensibilisant*** | | | | |
| Pd-Bphéide a | 5 mg | 0,5 % p/p | PA | |

| ***Autres composants*** | | | | |
|---|---|---|---|---|
| Ethanol absolu | 40 µl | 4 % v/p | Co-solvant | PE 4^{ème} édition 1318, A2000 |
| Alcool benzylique | 10 µl | 1 % v/p | Co-solvant | PE 4^{ème} édition 0256, 1997 |
| Solutol HS 15 | 100 mg | 10 % p/p | Tensioactif | |
| NaOH 0,028 M | 500 µl | 50 % v/p | Agent salifiant | PE 4^{ème} édition 0677, 1997 |
| Acide citrique 0,01 M | QS 7,2±0,4 | QS 7,2±0,4 | Agent tampon | PE 4^{ème} édition 0456, 1997 |
| EPPI QS | 1 g | 100 | Diluant | PE 4^{ème} édition 0169, A2000 |

Cette Formulation 3 de la présente invention est limpide, fortement colorée (pourpre foncé) et elle ne contient aucune trace de précipité, ce qui montre que le Pd-Bphéide a est complètement solubilisé.

Le spectre d'absorption UV du Pd-Bphéide a dans cette Formulation 3 de la présente invention montrait 5 pics principaux à 344, 385, 538, 763 et 818 nm avec un rapport de DO 763/818 voisin de 1, indiquant que le monomère représente au niveau spectral 50 % du produit.

Selon les conditions ICH, cette Formulation 2 est stable à 4°C pendant au moins 6 mois, ceci étant vérifié par dosage HPLC et UV.

### Formulation injectable 4

Formulation 4 contenant 1 mg/ml de Pd-Bphéide a comme agent photosensibilisant

### Véhicule :

Phase organique : alcool benzylique/éthanol (20/80) 5 % v/p
Tensio-actif : Crémophore ®EL 5 % p/p
NaOH : 0,007 M
pH ajusté à 7,2 ± 0,4 par l'acide citrique 0,01 M

La formulation 4 a été préparée comme suit :

Dans un récipient en verre de dimension convenable contenant 1 mg de Pd-Bphéide a préparé comme décrit ci-dessus, il a été ajouté 50 µl d'un mélange 20:80 V/V d'alcool benzylique et d'éthanol et le mélange a été légèrement agité. Ensuite, il a été ajouté au mélange 50 mg de Crémophore®EL et le mélange a été agité au vortex. Ensuite, il a été ajouté au mélange 500 µl d'une solution d'hydroxyde de sodium NaOH à 0,007 M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la formulation 4 est représentée sur le Tableau 4 suivant :

**Tableau 4**

| Nom des composants | Formule | | Fonctions | Référence aux normes |
|---|---|---|---|---|
| | unitaire | centésimale (%) | | |
| ***Agent photosensibilisant*** | | | | |
| Pd-Bphéide a | 1 mg | 0,1 % p/p | PA | |

| ***Autres composants*** | | | | |
|---|---|---|---|---|
| Ethanol absolu | 40 µl | 4 % v/p | Co-solvant | PE 4^{ème} édition 1318, A2000 |
| Alcool benzylique | 10 µl | 1 % v/p | Co-solvant | PE 4^{ème} édition 0256, 1997 |
| Crémophore®EL | 50 mg | 5 % p/p | Tensioactif | PE 4^{ème} édition 1082, 1997 |
| NaOH 0,007 M | 500 µl | 50 % v/p | Agent salifiant | PE 4^{ème} édition 0677, 1997 |
| Acide citrique 0,01 M | QS 7,2 ± 0,4 | QS 7,2 ± 0,4 | Agent tampon | PE 4^{ème} édition 0456, 1997 |
| EPPI QS | 1 g | 100 | Diluant | PE 4^{ème} édition 0169, A2000 |

Cette Formulation 4 de la présente invention est limpide, fortement colorée (pourpre foncé) et elle ne contient aucune trace de précipité, ce qui montre que le Pd-Bphéide a est complètement solubilisé.

Le spectre d'absorption UV du Pd-Bphéide a dans cette Formulation 4 de la présente invention montrait 5 pics principaux à 344, 385, 538, 763 et 818 nm avec un rapport de DO 763/818 de 80/20, indiquant que le monomère représente au niveau spectral 80 % du produit.

Selon les conditions ICH, cette Formulation 4 est stable à 4°C pendant au moins 6 mois, ceci étant vérifié par dosage HPLC et UV.

### Formulation injectable 5

Formulation 5 contenant 1 mg/ml de Pd-Bphéide a comme agent photosensibilisant

### Véhicule en phase aqueuse :

Phase organique : propylène glycol 5 % v/p
Tensio-actif : Crémophore ®EL 5 % p/p
NaOH : 0,007 M
pH ajusté à 7,2 ± 0,4 par l'acide citrique 0,01 M

La formulation 5 a été préparée comme suit :

Dans un récipient en verre de dimension convenable contenant 1 mg de Pd-Bphéide a préparé comme ci-dessus, il a été ajouté 50 µl de propylène glycol et le mélange a été légèrement agité. Ensuite, il a été ajouté au mélange 50 mg de Crémophore ®EL et le mélange a été agité au vortex. Ensuite, il a été ajouté au mélange 500 µl d'une solution d'hydroxyde de sodium NaOH à 0,007 M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la Formulation 5 est représentée sur le Tableau 5 suivant :

**Tableau 5**

| Nom des composants | Formule | | Fonctions | Référence aux normes |
|---|---|---|---|---|
| | unitaire | centésimale (%) | | |
| ***Agent photosensibilisant*** | | | | |
| Pd-Bphéide a | 1 mg | 0,1 % p/p | PA | |

| ***Autres composants*** | | | | |
|---|---|---|---|---|
| Propylène glycol | 50 µl | 5 % v/p | Co-solvant | PE 4^{ème} édition 0430, 1997 |
| Crémophore®EL | 50 mg | 5 % p/p | Tensioactif | PE 4^{ème} édition 1082, 1997 |
| NaOH 0,007 M | 500 µl | 50 % v/p | Agent salifiant | PE 4^{ème} édition 0677, 1997 |
| Acide citrique 0,01 M | QS 7,2 ± 0,4 | QS 7,2 ± 0,4 | Agent tampon | PE 4^{ème} édition 0456, 1997 |
| EPPI QS | 1 g | 100 | Diluant | PE 4^{ème} édition 0169,A2000 |

Cette Formulation 5 de la présente invention est limpide, fortement colorée (pourpre foncé) et elle ne contient aucune trace de précipité, ce qui montre que le Pd-Bphéide a est complètement solubilisé.

Le spectre d'absorption UV du Pd-Bphéide a dans cette Formulation 5 de la présente invention montrait 5 pics principaux à 344, 385, 538, 763 et 818 nm avec un rapport de DO 763/818 de 60/40, indiquant que le monomère représente au niveau spectral 60 % du produit.

Selon les conditions ICH, cette Formulation 5 est stable à 4°C pendant au moins 6 mois, ceci étant vérifié par dosage HPLC et UV.

### Formulation Comparative 1

Formulation Comparative 1 contenant 1 mg/ml de Pd-Bphéide a

### Véhicule :

Tensioactif : Crémophore ® EL 5 % p/p
NaOH : 0,007 M
pH ajusté à 7,2 ± 0,4 par l'acide citrique 0,01 M

La Formulation Comparative 1a été préparée comme suit :

Dans un récipient en verre de dimension appropriée contenant 1 mg de Pd-Bphéide a préparé comme décrit ci-dessus, il a été ajouté 50 mg de Crémophore ®EL et le mélange a été agité au vortex. Ensuite, il a été ajouté au mélange 500 µl d'une solution d'hydroxyde de sodium NaOH à 0,007 M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la Formulation Comparative 1 est représentée sur le Tableau 6 suivant :

**Tableau 6**

| Nom des composants | Formule | | Fonctions | Référence aux normes |
|---|---|---|---|---|
| | unitaire | centésimale (%) | | |
| Pd-Bphéide a | 1 mg | 0,1 % p/p | PA | |
| Crémophore®EL | 50 mg | 5 % p/p | Tensioactif | PE 4^{ème} édition 1082, 1997 |
| NaOH 0,007 M | 500 µl | 50 % v/p | Agent salifiant | PE 4^{ème} édition 0677, 1997 |
| Acide citrique 0,01 M | QS 7,2 ± 0,4 | QS 7,2 ± 0.4 | Agent tampon | PE 4^{ème} édition 0456, 1997 |
| EPPI QS | 1 g | 100 | Diluant | PE 4^{ème} édition 0169,A2000 |

Cette Formulation Comparative 1 contient un précipité, ce qui montre bien que le Pd-Bphéide a n'est pas complètement solubilisé, en l'absence du mélange alcool benzylique/éthanol ou du propylène glycol.

### Formulation Comparative 2

Formulation Comparative 2 contenant 1 mg/ml de Pd-Bphéide a

### Véhicule :

Ethanol 5 %
Tensioactif : Crémophore ®EL 5 % p/p
NaOH : 0,007 M
pH ajusté à 7,2 ± 0,4

La Formulation Comparative 2 a été préparée comme suit :

Dans un récipient en verre de dimension convenable contenant 1 mg de Pd-Bphéide a préparé comme décrit ci-dessus, il a été ajouté au mélange 50 µl d'éthanol et le mélange a été légèrement agité. Ensuite, il a été ajouté 50 mg de Crémophore®EL et le mélange a été agité au vortex. Ensuite, il a été ajouté au mélange 500 µl d'une solution d'hydroxyde de sodium NaOH à 0,007 M et le mélange a été agité quelques secondes au vortex. Le mélange a été ensuite placé sous ultrasons pendant 30 minutes (température < 25°C) en agitant de temps en temps à la main. Ensuite, le pH a été ajusté à 7,2 ± 0,4 avec de l'acide citrique 0,01 M et le mélange a été complété à 1 g avec de l'eau pour préparation injectable (EPPI).

La composition exacte de la Formulation Comparative 2 est représentée sur le Tableau 7 suivant :

**Tableau 7**

| Nom des composants | unitaire | centésimale (%) | Fonctions | Référence aux normes |
|---|---|---|---|---|
| Pd-Bphéide a | 1 mg | 0,1 % p/p | PA | |
| Ethanol | 50 µl | 5 % v/p | co-solvant | PE 4^{ème} édition 1318, A 2000 |
| Crémophore®EL | 50 mg | 5 % p/p | Tensioactif | PE 4^{ème} édition 1082, 1997 |
| NaOH 0,007 M | 500 µl | 50 % v/p | Agent salifiant | PE 4^{ème} édition 0677, 1997 |
| Acide citrique 0,01 M | QS 7,2 ± 0,4 | QS 7,2 ± 0,4 | Agent tampon | PE 4^{ème} édition 0456, 1997 |
| EPPI QS | 1 g | 100 | Diluant | PE 4^{ème} édition 0169,A2000 |

Cette Formulation Comparative 2 flocule, ce qui montre bien que le Pd-Bphéide a n'est pas complètement solubilisé, en l'absence d'alcool benzylique.

## Revendications

1. Formulation galénique injectable pour utilisation dans un diagnostic ou une thérapie photodynamique (PDT), ladite formulation galénique contenant :
- un composé représenté par la formule générale (I) suivante : 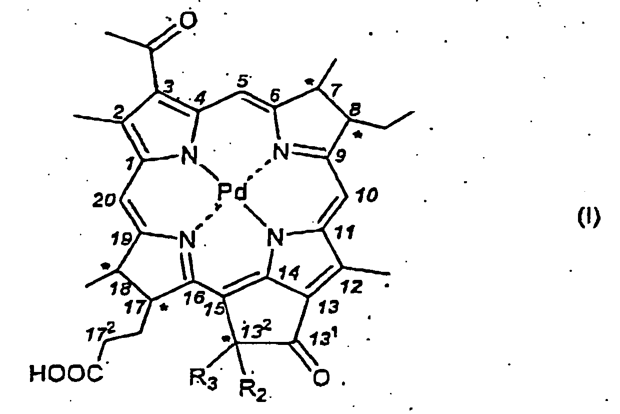 dans laquelle
R₂ représente un groupe H, OH ou COOR₄, où R₄ est un hydrogène ou un alkyle en C₁-C₁₂ ou un cycloalkyle en C₃-C₁₂,
R₃ représente H, OH, ou un alkyle ou alcoxy en C₁-C₁₂ et
* représente un carbone asymétrique
sous la forme d'un sel de métal alcalin, dans une quantité ne dépassant pas 10 mg/ml, en tant qu'agent photosensibilisant, et
- un véhicule en phase aqueuse pharmaceutiquement acceptable,
**caractérisée en ce que** le véhicule en phase aqueuse pharmaceutiquement acceptable contient au moins un mélange alcool benzylique - éthanol ayant un rapport de mélange de 15 : 85 à 25 : 75 ou du propylène glycol en tant qu'agent de solubilisation du photosensibilisant et un tensioactif permettant de diminuer l'agrégation du photosensibilisant, ledit agent tensioactif étant contenu dans une quantité ne dépassant pas 20 % en poids par rapport au poids total de la formulation, avec la condition que lorsque le tensioactif est un crémophore, sa quantité ne doit pas dépasser 5 % en poids par rapport au poids total de la formulation,

2. Formulation injectable selon la revendication 1, **caractérisée en ce que** le l'agent photosensibilisant de Formule (I) est le palladium-bactériophéophorbide a (Pd-Bphéide a) représenté par la Formule (II) suivante: 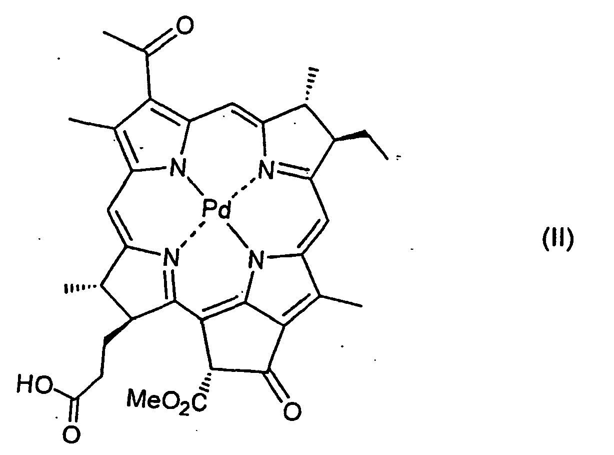 sous la forme de sel de métal alcalin.

3. Formulation galénique selon la revendication 1 ou 2, **caractérisée en ce que** le composé de Formule (I) ou le composé de Formule (II) est sous forme de sel de sodium.

4. Formulation injectable selon l'une quelconque des revendications 1 à 3, **caractérisée en ce que** l'agent de solubilisation est un mélange alcool benzyliqueéthanol contenu dans la formulation dans une quantité ne dépassant pas 5 % en volume par rapport à la masse totale de la formulation.

5. Formulation galénique injectable selon l'une quelconque des revendications 1 à 4, **caractérisée en ce que** l'agent de solubilisation est un mélange alcool benzylique-éthanol ayant un rapport de mélange de 20 : 80 v/v.

6. Formulation injectable selon l'une quelconque des revendications 1 à 3, **caractérisée en ce que** l'agent de solubilisation est le propylène glycol contenu dans la formulation dans une quantité ne dépassant pas 5 % en volume par rapport à la masse totale de la formulation.

7. Formulation injectable selon l'une quelconque des revendications 1 à 6, **caractérisée en ce que** l'agent tensioactif est le Crémophore ® EL, le Crémophore ® EL P ou le Solutol HS 15™.

8. Formulation galénique injectable selon l'une quelconque des revendications 1 à 7, **caractérisée en ce qu'**elle contient, en tant qu'agent tensioactif, du Crémophore ® EL ou du Crémophore ® EL P dans une quantité ne dépassant pas 5 % en masse par rapport à la masse totale de la formulation.

9. Procédé de préparation d'une formulation galénique injectable pour utilisation dans un diagnostic ou une thérapie photodynamique (PDT) contenant un composé représenté par la Formule (I) suivante : 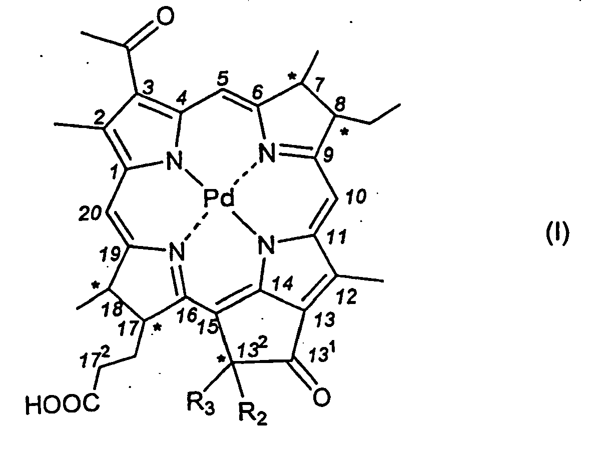 dans laquelle
R₂ représente un groupe H, OH ou COOR₄, où R₄ est un hydrogène ou un alkyle en C₁-C₁₂ ou un cycloalkyle en C₃-C₁₂,
R₃ représente H, OH, ou un alkyle ou alcoxy en C₁-C₁₂ et
* représente un carbone asymétrique
sous la forme de sel de métal alcalin, dans une quantité allant jusqu'à 10 mg/ml en tant qu'agent photosensibilisant et un véhicule en phase aqueuse pharmaceutiquement acceptable, ledit procédé comprenant, dans l'ordre indiqué, les étapes consistant à :
(1) mouiller l'agent photosensibilisant de Formule (I) sous sa forme acide avec un agent de solubilisation constitué d'un mélange alcool benzylique - éthanol dans un rapport de mélange de 15 : 85 à 25 : 75 ou de propylène glycol;
(2) ajouter un agent tensioactif dans une quantité ne dépassant pas 20 % en poids par rapport au poids final de la formulation;
(3) ajouter un hydroxyde de métal alcalin dans une quantité suffisante pour neutraliser la fonction acide de l'agent photosensibilisant de Formule (I);
(4) agiter le mélange en phase aqueuse de manière suffisante pour permettre la salification complète de l'agent photosensibilisant et sa totale solubilisation;
(5) ajouter un agent tampon destiné à ajuster le pH à une valeur physiologiquement acceptable de 7,2 ± 0,4;
(6) si nécessaire, ajuster le volume de la formulation avec de l'eau pour préparation injectable.

10. Procédé de préparation d'une formulation galénique injectable selon la revendication 9, **caractérisé en ce que** l'agent photosensibilisant de Formule (I) sous sa forme acide est le palladium-bactériophéorphorbide a (Pd-Bphéide a) représenté par la Formule (II) suivante :

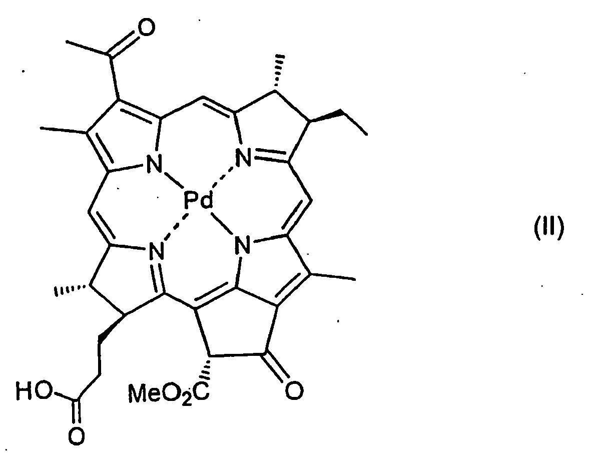

11. Procédé de préparation d'une formulation galénique injectable selon la revendication 9 ou 10, **caractérisé en ce que** l'agent de solubilisation est ajouté dans une quantité ne dépassant pas 5 % en volume par rapport à la masse totale de la solution.

12. Procédé de préparation d'une formulation galénique injectable selon l'une quelconque des revendications 9 à 11, **caractérisé en ce que** l'agent de solubilisation ajouté est un mélange alcool benzylique-éthanol ayant un rapport de mélange de 20 : 80.

13. Procédé de préparation d'une formulation galénique injectable selon l'une quelconque des revendications 9 à 12, **caractérisé en ce que** l'agent tensioactif ajouté est le Crémophore ® EL, le Crémophore ® EL P ou le Solutol HS 15 ™.

14. Procédé de préparation d'une formulation galénique injectable selon l'une quelconque des revendications 9 à 13, **caractérisé en ce que** l'agent tensioactif ajouté est le Crémophore ® EL ou le Crémophore ® EL P et **en ce qu'**il est ajouté dans une quantité ne dépassant pas 5 % en masse par rapport à la masse totale de la formulation.

15. Procédé de préparation d'une formulation galénique injectable selon l'une quelconque des revendications 9 à 14, **caractérisé en ce que** l'hydroxyde de métal alcalin est l'hydroxyde de sodium.

16. Procédé de préparation d'une formulation galénique injectable selon l'une quelconque des revendications 9 à 15, **caractérisé en ce que** l'agent tampon destiné à ajuster le pH ajouté est l'acide citrique.

17. Procédé de préparation d'une formulation galénique injectable selon l'une quelconque des revendications 9 à 16, **caractérisé en ce que** les étapes (1) à (6) sont effectuées sous atmosphère de gaz inerte.

18. Formulation galénique injectable obtenue par le procédé selon l'une quelconque des revendications 9 à 17.

## Claims

1. An injectable galenic formulation for use in a diagnosis or in a photodynamic therapy (PDT), said galenic formulation containing :
- a compound represented by the following general formula (I) : 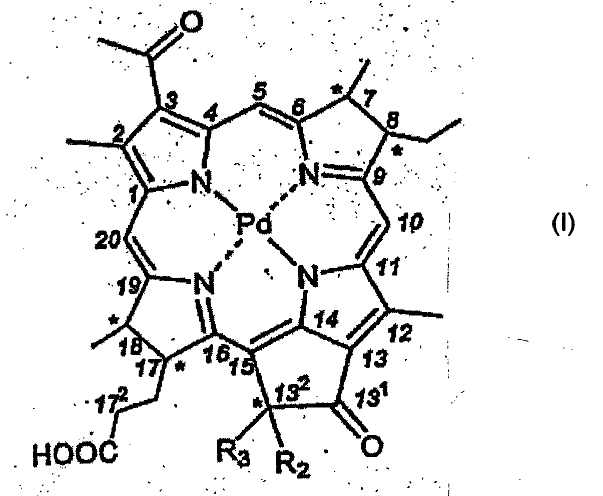 in which
R₂ represents a group H, OH or COOR₄, wherein R₄ is a hydrogen or a C₁ - C₁₂ alkyl or a C₃ - C₁₂ cycloalkyl,
R₃ represents H, OH or a C₁ - C₁₂ alkyl or alcoxy and
* represents an asymmetric carbon,
in the form of a salt of an alkaline metal, in an amount not exceeding 10 mg / ml, as photosensitising agent, and
- a pharmaceutically acceptable carrier in an aqueous phase,
**characterised in that** the pharmaceutically acceptable carrier in an aqueous phase contains at least a mixture of benzyl alcohol - ethanol in a ratio from 15 : 85 to 25 : 75 or propylene glycol, acting as a solubilizing agent for the photosensitising agent and a surfactant capable of decreasing the aggregation of the photosensitising agent, said surfactant provided in an amount not exceeding 20 wt. % relative to the total weight of the formulation, with the proviso that when the surfactant is Cremophore, its amount does not exceed 5 wt. % relative to the total weight of the formulation.

2. An injectable formulation according to claim 1, wherein the photosensitising agent of Formula (I) is palladium-bacteriopheophorbide a (Pd-Bpheide a) represented by the following Formula (II) : 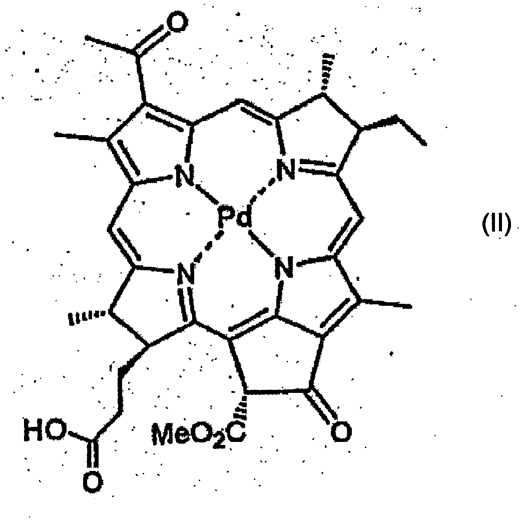 in the form of a salt of an alkaline metal.

3. A galenic formulation according to claim 1, wherein the compound of Formula (I) or the compound of Formula (II) is in the form of the sodium salt.

4. An injectable formulation according to claim 1, wherein the solubilizing agent is a mixture of benzyl alcohol - ethanol contained in the formulation in an amount not exceeding 5 vol. % relative to the total weight of the formulation.

5. An injectable galenic formulation according to claim 1, wherein the solubilizing agent is a mixture of benzyl alcohol - ethanol in a ratio 20 : 80 vol. / vol.

6. An injectable formulation according to claim 1, wherein the solubilizing agent is propylene glycol, contained in the formulation in an amount not exceeding 5 vol. % relative to the total weight of the formulation.

7. An injectable formulation according to claim 1, wherein the surfactant is Cremophore ® EL, Cremophore ® EL P or Solutol HS 15 ™.

8. An injectable galenic formulation according to claim 1, wherein it contains, as the surfactant, Cremophore ® EL or Cremophore ® EL P in an amount not exceeding 5 wt. % relative to the total weight of the formulation.

9. A method for the preparation of an injectable galenic formulation for use in a diagnosis or in a photodynamic therapy (PDT) containing a compound represented by the following Formula (I) : 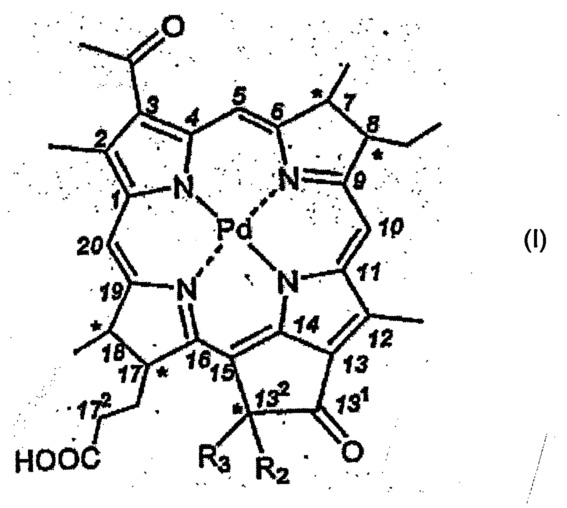 in which
R₂ represents a group H, OH or COOR₄, wherein R₄ is a hydrogen or a C₁- C₁₂ alkyl or a C₃- C₁₂ cycloalkyl,
R₃ represents H, OH or a C₁- C₁₂ alkyl or alcoxy and
* represents an asymmetric carbon,
in the form of a salt of an alkaline metal, in a amount of up to 10 mg / ml, as the photosensitising agent and a pharmaceutically acceptable carrier in an aqueous phase, said method including, in the order indicated, the steps consisting in :
(1) wetting the photosensitising agent of Formula (I) in its acid form with a solubilizing agent comprised of a mixture of benzyl alcohol - ethanol in a ratio from 15 : 85 to 25 : 75, or of propylene glycol ;
(2) adding a surfactant in an amount not exceeding 20 wt. % relative to the final weight of the formulation ;
(3) adding a hydroxide of an alkaline metal in an amount sufficient for neutralising the acid function of the photosensitising agent of Formula (I) ;
(4) stirring the mixture in the aqueous phase sufficiently to ensure a complete conversion of the photosensitising agent into the salt thereof and its complete solubilization ;
(5) adding a buffering agent designed for adjusting the pH to a physiologically acceptable value of 7.2 ± 0.4 ;
(6) if necessary, adjusting the volume of the formulation with water for injectable preparations.

10. A method for the preparation of an injectable galenic formulation according to claim 9, wherein the photosensitising agent of Formula (I) in its acid form is palladium-bacteriopheorphorbide a (Pd-Bpheide a) represented by the following Formula (II) :

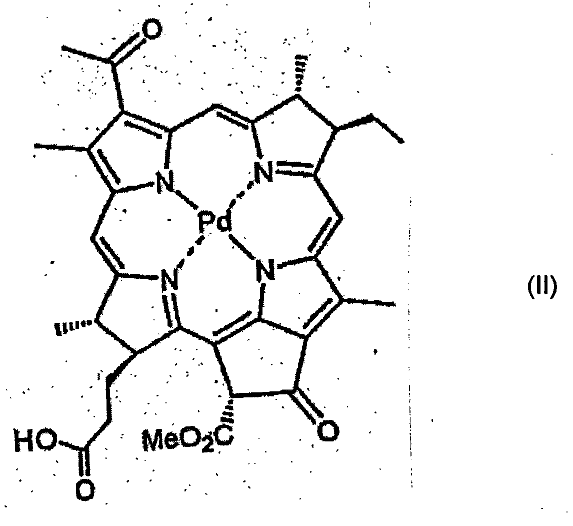

11. A method for the preparation of an injectable galenic formulation according to claim 9, **characterised in** the solubilizing agent is added in an amount not exceeding 5 vol. % relative to the total weight of the solution.

12. A method for the preparation of an injectable galenic formulation according to claim 9, wherein the solubilizing agent added is a mixture of benzyl alcohol - ethanol in a ratio of 20 : 80.

13. A method for the preparation of an injectable galenic formulation according to claim 9, wherein the surfactant added is Cremophore ® EL or Cremophore ® EL P or Solutol HS 15 ^{™}.

14. A method for the preparation of an injectable galenic formulation according to claim 9, wherein the surfactant added is Cremophore ® EL or Cremophore ® EL P and in that it is added in an amount not exceeding 5 wt. % relative to the total weight of the formulation.

15. A method for the preparation of an injectable galenic formulation according to claim 9, wherein the hydroxide of an alkaline metal is sodium hydroxide.

16. A method for the preparation of an injectable galenic formulation according to claim 9, wherein the buffering agent added for adjusting the pH is citric acid.

17. A method for the preparation of an injectable galenic formulation according to claim 9, wherein the steps (1) to (6) are carried out in an atmosphere of an inert gas.

18. An injectable galenic formulation obtained by the method according to claim 9.

## Patentansprüche

1. Injizierbare galenische Formulierung zur Verwendung in einer Diagnose oder einer photodynamischen Therapie (PDT), wobei die galenische Formulierung:
- eine durch die folgende allgemeine Formel (I) dargestellte Verbindung: 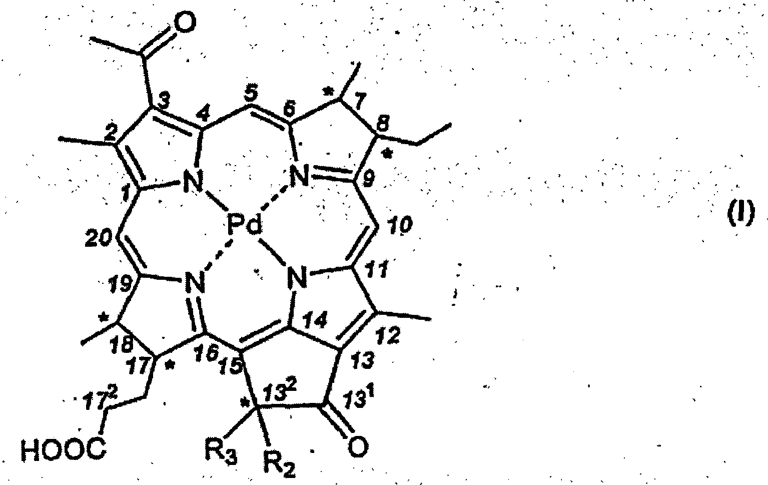 in der
R₂ eine H-, OH- oder COOR₄-Gruppe darstellt, wo R₄ ein Wasserstoff oder ein C₁- bis C₁₂-Alkyl oder ein C₃- bis C₁₂-Cycloalkyl ist,
R₃ H, OH oder ein C₁- bis C₁₂-Alkyl oder-Alkoxy darstellt und
* einen asymmetrischen Kohlenstoff darstellt,
in Gestalt eines Alkalimetallsalzes in einer 10 mg/ml nicht überschreitenden Menge als Photosensibilisator und
- einen pharmazeutisch annehmbaren Träger in wässriger Phase enthält,
**dadurch gekennzeichnet, dass** der pharmazeutisch annehmbare Träger in wässriger Phase zumindest eine Mischung aus Benzylalkohol und Ethanol in einem Mischungsverhältnis von 15 : 85 bis 25 : 75 oder Propylenglykol als Solubilisator des Photosensibilisators sowie ein Tensid enthält, das die Aggregation des Photosensibilisators zu verringern gestattet, wobei das Tensid in einer Menge enthalten ist, die 20 Gewichtsprozent, bezogen auf das Gesamtgewicht der Formulierung, nicht übersteigt, unter der Bedingung, dass die Menge des Tensids, wenn es ein Cremophor ist, 5 Gewichtsprozent, bezogen auf das Gesamtgewicht der Formulierung, nicht übersteigen darf.

2. Injizierbare Formulierung nach Anspruch 1, **dadurch gekennzeichnet, dass** der Photosensibilisator der Formel (I) das durch die folgende Formel (II) dargestellte Palladium-Bakteriopheophorbid a (Pd-BPheid a): 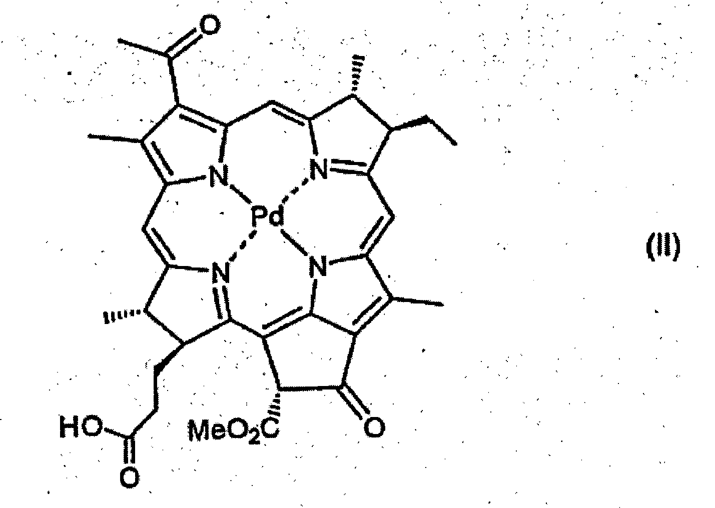 in Gestalt eines Alkalimetallsalzes ist.

3. Galenische Formulierung nach Anspruch 1 oder 2, **dadurch gekennzeichnet, dass** die Verbindung der Formel (I) oder die Verbindung der Formel (II) in Gestalt des Natriumsalzes vorliegt.

4. Injizierbare Formulierung nach einem der Ansprüche 1 bis 3, **dadurch gekennzeichnet, dass** der Solubilisator eine Mischung aus Benzylalkohol und Ethanol ist, die in der Formulierung in einer Menge enthalten ist, die 5 Volumenprozent, bezogen auf die Gesamtmasse der Formulierung, nicht übersteigt.

5. Injizierbare galenische Formulierung nach einem der Ansprüche 1 bis 4, **dadurch gekennzeichnet, dass** der Solubilisator eine Mischung aus Benzylalkohol und Ethanol ist, die ein Mischungsverhältnis von 20 : 80 (v/v) besitzt.

6. Injizierbare Formulierung nach einem der Ansprüche 1 bis 3, **dadurch gekennzeichnet, dass** der Solubilisator Propylenglykol ist, das in der Formulierung in einer Menge enthalten ist, die 5 Volumenprozent, bezogen auf die Gesamtmasse der Formulierung, nicht übersteigt.

7. Injizierbare Formulierung nach einem der Ansprüche 1 bis 6, **dadurch gekennzeichnet, dass** das Tensid Cremophor® EL, Cremophor® EL P oder Solutol HS 15™ ist.

8. Injizierbare galenische Formulierung nach einem der Ansprüche 1 bis 7, **dadurch gekennzeichnet, dass** sie als Tensid Cremophor® EL oder Cremophor® EL P in einer Menge enthält, die 5 Masseprozent, bezogen auf die Gesamtmasse der Formulierung, nicht übersteigt.

9. Verfahren zur Herstellung einer injizierbaren galenischen Formulierung zur Verwendung in einer Diagnose oder einer photodynamischen Therapie (PDT), die eine durch die folgende Formel (I) dargestellte Verbindung: 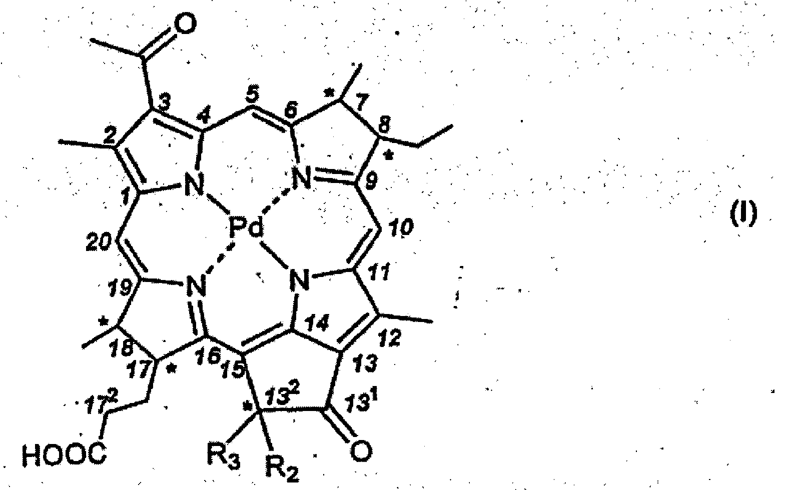 in der
R₂ eine H-, OH- oder COOR₄-Gruppe darstellt, wo R₄ ein Wasserstoff oder ein C₁- bis C₁₂-Alkyl oder ein C₃- bis C₁₂-Cycloalkyl ist,
R₃ H, OH oder ein C₁- bis C₁₂-Alkyl oder -Alkoxy darstellt und
* einen asymmetrischen Kohlenstoff darstellt,
in Gestalt eines Alkalimetallsalzes in einer bis zu 10 mg/ml reichenden Menge als Photosensibilisator und einen pharmazeutisch annehmbaren Träger in wässriger Phase enthält, wobei das Verfahren in der angezeigten Reihenfolge die Schritte umfasst, die daraus bestehen:
1) den Photosensibilisator der Formel (I) in seiner Säureform mit einem Solubilisator zu benetzen, der aus einer Mischung von Benzylalkohol und Ethanol in einem Mischungsverhältnis von 15 : 85 bis 25 : 75 oder aus Propylenglykol besteht;
2) ein Tensid in einer Menge hinzuzufügen, die 20 Gewichtsprozent, bezogen auf das schlussendliche Gewicht der Formulierung, nicht übersteigt;
3) ein Alkalimetallhydroxid in einer Menge hinzuzugeben, die genügt, um die Säurefunktion des Photosensibilisators der Formel (I) zu neutralisieren;
4) die Mischung in wässriger Phase genügend zu rühren, um die vollständige Salzbildung des Photosensibilisators und seine vollkommene Solubilisierung zu ermöglichen;
5) einen Pufferstoff hinzuzufügen, der dafür bestimmt ist, den pH-Wert auf einen physiologisch annehmbaren Wert von 7,2 ± 0,4 einzustellen;
6) wenn erforderlich, mit Wasser das Volumen der Formulierung für eine injizierbare Zubereitung einzustellen.

10. Verfahren zur Herstellung einer injizierbaren galenischen Formulierung nach Anspruch 9, **dadurch gekennzeichnet, dass** der Photosensibilisator der Formel (I) in seiner Säureform das durch die folgende Formel (II) dargestellte Palladium-Bakteriopheophorbid a (Pd-BPheid a) ist:

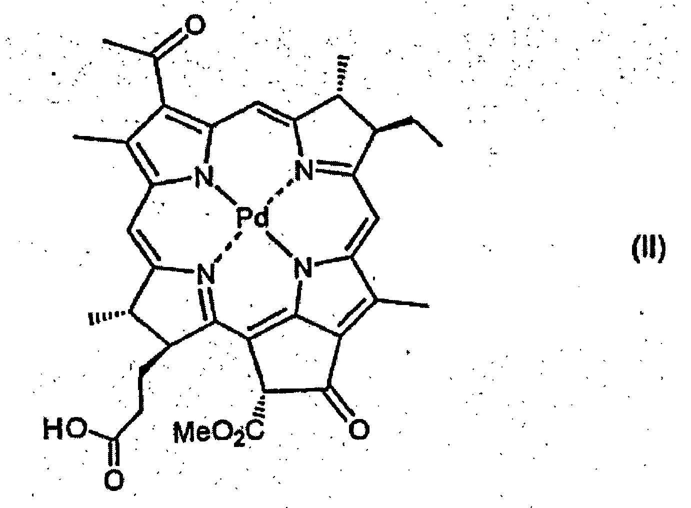

11. Verfahren zur Herstellung einer injizierbaren galenischen Formulierung nach Anspruch 9 oder 10, **dadurch gekennzeichnet, dass** der Solubilisator in einer Menge hinzugefügt wird, die 5 Volumenprozent, bezogen auf die Gesamtmasse der Lösung, nicht übersteigt.

12. Verfahren zur Herstellung einer injizierbaren galenischen Formulierung nach einem der Ansprüche 9 bis 11, **dadurch gekennzeichnet, dass** der hinzugefügte Solubilisator eine Mischung aus Benzylalkohol und Ethanol ist, die ein Mischungsverhältnis von 20 : 80 (v/v) besitzt.

13. Verfahren zur Herstellung einer injizierbaren galenischen Formulierung nach einem der Ansprüche 9 bis 12, **dadurch gekennzeichnet, dass** das hinzugefügte Tensid Cremophor® EL, Cremophor® EL P oder Solutol HS 15™ ist.

14. Verfahren zur Herstellung einer injizierbaren galenischen Formulierung nach einem der Ansprüche 9 bis 13, **dadurch gekennzeichnet, dass** das hinzugefügte Tensid Cremophor® EL oder Cremophor® EL P ist und dass es in einer Menge hinzugefügt wird, die 5 Masseprozent, bezogen auf die Gesamtmasse der Formulierung, nicht übersteigt.

15. Verfahren zur Herstellung einer injizierbaren galenischen Formulierung nach einem der Ansprüche 9 bis 14, **dadurch gekennzeichnet, dass** das Alkalimetallhydroxid Natriumhydroxid ist.

16. Verfahren zur Herstellung einer injizierbaren galenischen Formulierung nach einem der Ansprüche 9 bis 15, **dadurch gekennzeichnet, dass** der zur Einstellung des pH-Wertes bestimmte Pufferstoff Citronensäure ist.

17. Verfahren zur Herstellung einer injizierbaren galenischen Formulierung nach einem der Ansprüche 9 bis 16, **dadurch gekennzeichnet, dass** die Schritte (1) bis (6) in einer Inertgasatmosphäre ausgeführt werden.

18. Injizierbare galenische Formulierung, gewonnen durch das Verfahren nach einem beliebigen der Ansprüche 9 bis 17.
